# EUROPÄISCHE PATENTANMELDUNG

(11) **EP 1 541 187 A1**
(43) Veröffentlichungstag der Anmeldung: **15.06.2005**
(21) Anmeldenummer: 04405733.9
(22) Anmeldetag: 25.11.2004
(51) Int. Cl.: A61M 16/04

(54) **Lätzchen zum Schütze und zur diskreten Abdeckung des Tracheostomas**

(30) Priorität: 11.12.2003 CH 21102003
(71) Anmelder: RIEDI-JOKS, Susanne, CH-6006 Luzern (CH)
(72) Erfinder: RIEDI-JOKS, Susanne, CH-6006 Luzern (CH)

(57) **Zusammenfassung**

Das Lätzchen (1) zum Schutze und zur diskreten Abdeckung des Trachoestomas mit einer angebrachten auswechselbaren Tasche (7) zur Aufnahme der zum Sprechen notwendigen Haltevorrichtung (8) und Kassette (9) bewirkt, dass die Haltevorrichtung (8) nicht mehr als Pflaster auf die Haut aufgeklebt werden muss, sondern in die Tasche (7) eingeschoben wird, womit die Reizung und das Wundwerden der Haut vermieden wird. Die Haltevorrichtung (8) und die Kassette (9) können, entgegen der bisherigen Anwendung, als Einwegartikel leicht gereinigt und wieder verwendet werden. Ausgehustetes Sekret kann leicht und diskret weggewischt werden. Das Lätzchen (1) mit der Tasche (7) können lose getragen werden und bestehen aus hautfreundlichen und kleider- und wäscheschonenden Textilgeweben (4, 10, 11, 14, 15, 19).

## Beschreibung

Die vorliegende Erfindung betrifft ein Lätzchen zum Schutze und zur diskreten Abdeckung des Trachoestomas mit einer auf der Körperseite auf die Lage des Tracheostomas einstellbaren Halterung angebrachten auswechselbaren Tasche zur Aufnahme einer Haltevorrichtung für die Fixierung einer Kassette mit einem Deckplatte-Feder-Ventilmechanismus für den zum Sprechen notwendig manuell zu betätigenden Tracheostomaverschluss.

Bei den Personen mit totaler Laryngektomie wurde bei der Operation der Kehlkopf zusammen mit den Stimmbändern entfernt, sodass diese Personen ohne Hilfsmittel nicht mehr in der Lage sind, sich lautsprachlich zu verständigen. Die Personen mit totaler Laryngektomie atmen nicht mehr durch die Nase, sondern durch ein bei der Operation am Hals geschaffenes Tracheostoma. Bei der Operation werden Luftwege von der Mundhöhle und der Speiseröhre getrennt. Die Luftröhre endet somit mit einer besonderen Öffnung im Hals, dem sogenannten Tracheostoma, nach aussen.

Damit sich die Personen mit totaler Laryngektomie wieder lautsprachlich verständigen können, bedürfen sie zur Wiederherstellung der Stimme eine Stimmprothese, die in die bei der Operation geschaffene Verbindung zwischen Speiseröhre und Luftröhre eingesetzt wird.

Damit eine Stimme mit der Stimmprothese erzeugt werden kann, muss das Tracheostoma beim Ausatmen verschlossen werden, und die ausgeatmete Luft wird so durch die Stimmprothese in die Speiseröhre geleitet, wo die Schleimhäute zu vibrieren beginnen und dabei einen Ton, respektive eine Stimme erzeugen.

Der Verschluss des Tracheostomas kann entweder mit einem Finger oder mit einen entsprechend geformten Gegenstand erfolgen, was aber bedeutet, dass das Tracheostoma offengelegt werden muss und daher nicht mehr geschützt, unhygienisch und indiskret gegenüber dem Gesprächspartner ist.

Der heute bekannte letzte Stand der Technik zum Verschliessen des Tracheostomas ist die Anwendung eines Pflasters mit einer in deren Mitte angeordneten Haltevorrichtung zur Aufnahme und Fixierung einer Kassette mit einem Deckplatte-Feder-Ventilmechanismus. Das Pflaster muss über das Tracheostoma geklebt werden und dicht mit der Haut abschliessen.

Die Erfahrung zeigt und man ist sich bewusst, dass durch das dauernde Tragen des aufgeklebten Pflasters sich die Haut rötet und entzündet, was praktisch bei Personen mit einer Pflasterallergie das Tragen des Pflasters zur unerträglichen Qual wird und daher dieses Pflaster von solchen Personen nicht angewendet werden kann.

Ein weiterer Nachteil bei der Verwendung des Pflasters ist, dass beim Aushusten von Sekret aus der Luftröhre die Entfernung des Sekretes schwierig ist und praktisch in der Öffentlichkeit nicht mehr diskret erfolgen kann und daher unmöglich ist.

In der Folge arbeitet sich das Sekret logischerweise unter dem Pflaster durch, was bewirkt, dass einerseits die Haftfähigkeit des Pflasters in dieser Zone stark beeinträchtigt und diese Region undicht wird und andererseits dieses ausgehustete Sekret eine Reizung und Rötung bis zur Wundwerdung der Haut verursacht.

Die Rötung und die Wundwerdung der Haut in der Umgebung des Tracheostomas muss mit einer Salbe behandelt werden, was aber wiederum bewirkt, dass die Haftfähigkeit des Pflaster auch nicht mehr gewährleistet ist.

Berücksichtigt man, dass das Pflaster wie die Kassette Einwegartikel sind und daher das Pflaster je nach Haftfähigkeit täglich oder alle zwei Tage und die Kassette mindestens einmal pro Tag ausgewechselt werden müssen.

Das sind Probleme, die gelöst werden müssen.

Es ist die Aufgabe der vorliegenden Erfindung, einen Gegenstand zu schaffen, der die erwähnten Probleme löst.

Die Erfindung löst diese Aufgabe mit einem Lätzchen, das sich dadurch auszeichnet, dass es mit einer auf der Körperseite auf die Lage des Tracheostomas mit individueller fein einstellbaren Halterung angebrachten auswechselbaren Tasche zur Aufnahme der Haltevorrichtung für die Fixierung der Kassette mit einem Deckplatte-Feder-Ventilmechanismus für den zum Sprechen notwendig manuell zu betätigenden Tracheostomaverschluss versehen ist.

In dieser Beschreibung, bei den Patentansprüchen, bei der Zusammenfassung und bei den Zeichnungen werden folgende Positionsbezeichnungen benützt.
Dabei bedeutet:
- 1: Lätzchen,
- 2: Festhalteverschluss am Lätzchen zum Verschliessen desselben beim Tragen durch Umschlingen um den Hals,
- 3: Gegenstück des Festhalteverschlusses 2,
- 4: hautfreundliches, steifes und luftdurchlässiges Textilgewebe,
- 5: Halterung am Lätzchen,
- 6: Gegenstück an der Tasche zur Halterung 5 am Lätzchen,
- 7: auswechselbar Tasche,
- 8: Haltevorrichtung ohne Klebstoff für die Fixierung der Kassette,
- 9: Kassette,
- 10: hautfreundliches, feines und weiches Textilgewebe beim Lätzchen,
- 11: Saum aus hautfreundlichem, feinem und weichem Textilgewebe,
- 12: dem Körper abgewandte Seite der auswechselbaren Tasche,
- 13: dem Körper zugewandte Seite der auswechselbaren Tasche,
- 14: hautfreundliches, feines und weiches Textilgewebe bei der auswechselbaren Tasche,
- 15: zusätzliches Textilgewebe bei der auswechselbaren Tasche,
- 16: Füllstoff als Polster bei der auswechselbaren Tasche,
- 17: Durchbruch bei der auswechselbaren Tasche,
- 18: Wulst am Rande des Durchbruches bei der auswechselbaren Tasche,
- 19: hautfreundliches, feines und weiches Textilgewebe beim Wulst,
- 20: weiches und elastisches Material.

Das Lätzchen 1 wird durch Umschlingen um den Hals getragen und mit einem auf den Körper, respektive auf den Hals fein einstellbaren Festhalteverschluss 2 am einen Kragenende und dem Gegenstück 3 des Festhalteverschlusses am anderen Kragenende fixiert.

Das Lätzchen 1 besteht aus mindestens einer Lage von hautfreundlichem, steifem und luftdurchlässige Textilgewebe 4, wobei sämtliche Partien, besonders diejenigen an den beiden Kragenenden, die direkt mit der Haut in Berührung kommen zur Vermeidung von Hautreizungen, Errötung und Wundwerdung der Haut und zum Schutze der darüber- und darunterliegenden Kleider und Wäschestücke aus einem hautfreundlichen, feinen und weichen Textilgewebe 10 hergestellt und die Ränder des hautfreundlichen steifen und luftdurchlässigen Textilgewebes 4 mit einem hautfreundlichen, feinen und weichen Textilgewebe 11 eingesäumt sind.

Am kragenähnlich ausgebildeten Saum 11 ist im Bereich der Vorderseite des Halses auf der der Haut zugewandten Innenseite des Lätzchens 1 die Halterung 5 und auf der der Haut abgewandeten Seite 12 der auswechselbaren Tasche 7 am oberen Rand das Gegenstück 6 der Halterung zur Fixierung der auswechselbaren Tasche 7 am Lätzchen 1 aufgenäht.

Die dem Körper abgewandte Seite 12 und die dem Körper zugewandte Seite 13 der auswechselbaren Tasche 7 besteht aus je einer Lage aus hautfreundlichem, feinem und weichem Textilgewebe 14 mit je einem der Form und der Grösse der Kassette 9 entsprechendem Durchbruch 17, damit die Kassette 9 auf den beiden Seiten 12 und 13 der auswechselbaren Tasche 7 herausragen kann.

Zur besseren Schonung der Haut in der Gegend des Tracheostomas wird die auswechselbare Tasche 7 auf der Inneseite der Seite 12 und der Seite 13 je mit einer zusätzlichen Lage eines Textilgewebes 15 versehen, wobei die dadurch entstehenden Zwischenräume mit einem Füllstoff 16 als Polster ausgefüllt werden, wobei die beiden zusätzlichen Lagen des Textilgewebes 15 und der Füllstoff 16 ebenfalls mit dem Durchbruch 17 zu versehen sind.

Bei der auswechselbaren Tasche 7 ist der Rand des Durchbruches 17 auf der der Haut zugewandten Seite 13 mit einem Wulst 18 aus einem weichen und elastischen Material 20, welcher mit einem hautfreundlichen, feinen und weichem Textilgewebe 19 eingesäumt ist, auszuführen.

Mit dem Wulst 18 wird beim Sprechen bei der manuell vorzunehmenden Betätigung der Kassette 9 eine bessere Abdichtung des Tracheostomas erreicht.

Die Haltevorrichtung 8 zur Aufnahme und Fixierung der Kassette 9 muss nicht mehr auf die Haut aufgeklebt werden, sondern kann mühelos und auf einfache Art in die auswechselbare Tasche 7 eingeschoben werden und bleibt auch infolge des Herausragens der Kassette 9 durch die Durchbrüche 17 in allen Richtungen unverrückbar fixiert.

Eine direkte Berührung des Klebstoffes des Pflasters an der Haltevorrichtung 8 mit der Haut findet nicht mehr statt und eine Reizung, Rötung oder Entzündung der Haut ist somit ausgeschlossen.

Ausgeworfenes Sekret aus dem Tracheostoma kann auf einfache Weise und diskret jederzeit mit einem Taschentuch weggewischt werden.

Das Lätzchen 1 mit der eingesetzten auswechselbaren Tasche 7, ausgerüstet mit der Haltevorrichtung 8 und der eingesetzten Kassette 9, wird bei uneingeschränkter Sprechfähigkeit und für die betreffende Person praktisch unbemerkbar wie ein Kleidungsstück lose auf dem Körper aufliegend getragen.

Während der Phase des Nichtsprechens ist die Abdichtung des Tracheostomas nicht nötig, wobei mit der Ausführung des Lätzchens 1 aus hautfreundlichem, steifem und luftdurchlässigem Textilgewebe 4, eingefasst mit dem Saum aus hautfreundlichem, feinem und weichem Textilgewebe 11, eine genügende Filterung der Atemluft und ein Wärme- und Feuchtigkeitsaustausch ebenfalls gewährleistet ist und bei der komplett bestückten auswechselbaren Tasche 7 mit der Haltevorrichtung 8 und der Kassette 9 die Atmung uneingeschränkt ist, kann auf den in der Kassette 9 eingesetzten Filter verzichtet werden.

Dies bedeutet, dass das Lätzchen 1, die auswechselbare Tasche 7, die Haltevorrichtung 8 und die Kassette 9 bei Verschmutzung gereinigt und gewaschen werden können, und daher, entgegen der bisherigen Anwendung der Haltevorrichtung 8, als ein auf die Haut aufklebbares Pflaster und die Kassette 9 mit dem eingesetzten Filter, als bisheriges Einwegmaterial, immer wieder verwendet werden können.

Es zeigt:

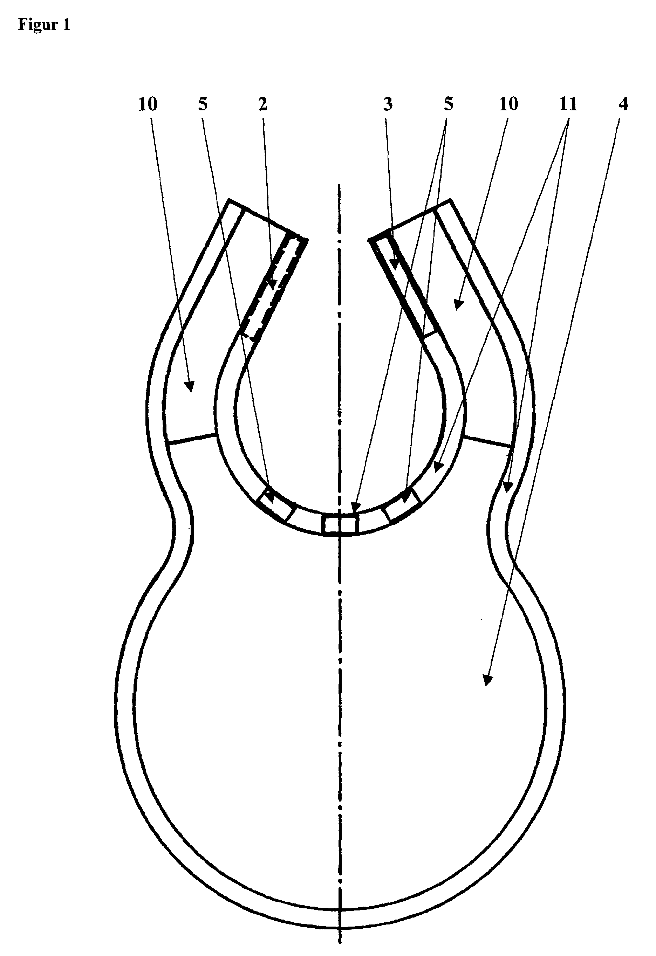

- Figur 1: den prinzipiellen Aufbau des Lätzchens 1 in der Ansicht von der dem Körper zugewandten Seite, ohne der auswechselbaren Tasche 7.

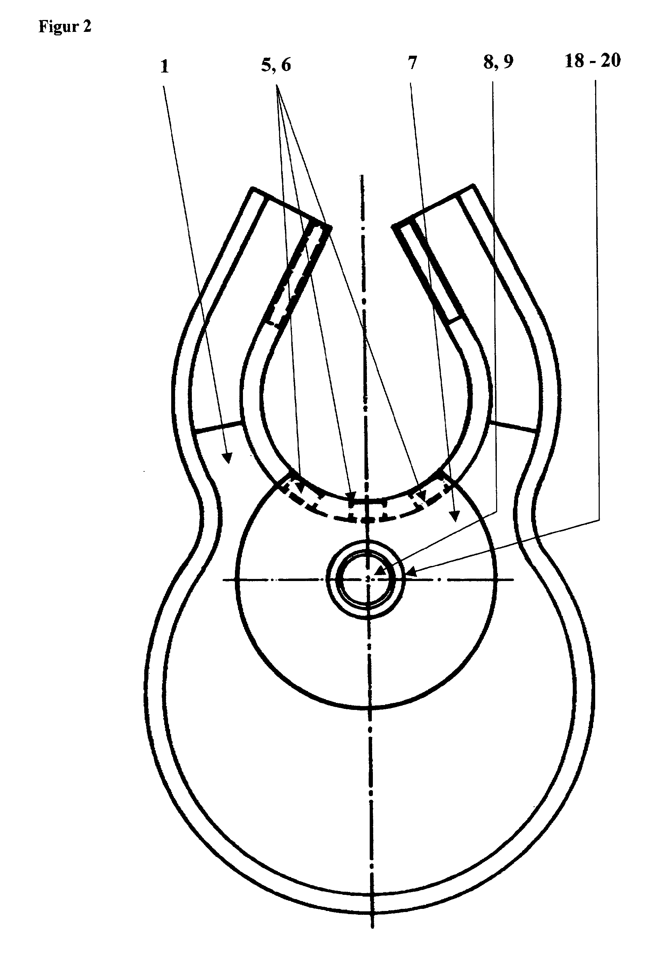

- Figur 2: den prinzipiellen Aufbau des Lätzchens 1 in der Ansicht von der dem Körper zugewandten Seite mit der mit der Haltevorrichtung 8 und der eingesetzten Kassette 9 bestückten, auswechselbaren Tasche 9.

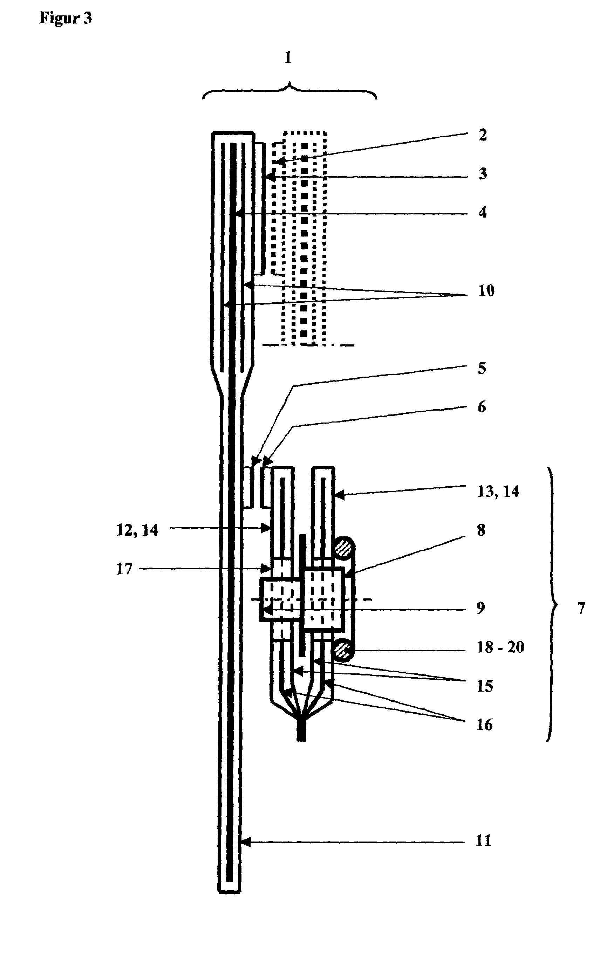

- Figur 3: den schematischen prinzipiellen Aufbau des Lätzchens 1 als Schnitt längs der senkrechten Symmetrieachse gemäss der Figur 2, als einzelne Schichten dargestellt.

## Patentansprüche

1. Lätzchen (1) zum Schutze und zur diskreten Abdeckung des Tracheostomas durch Umschlingen des menschlichen Halses mit einem individuell fein einstellbaren Festhalteverschluss (2 und 3), welches aus mindestens einer Lage hautfreundlichen, steifen und luftdurchlässigen Textilgewebe (4) besteht, **dadurch gekennzeichnet, dass** auf der Körperseite eine auf die Lage des Tracheostomas mit einer individuell fein einstellbaren Halterung (5 und 6) auswechselbare Tasche (7) mit einer dem Körper abgewandten Seite (12) und einer dem Körper zugewandten Seite (13) zur Aufnahme einer Haltevorrichtung (8) für die Fixierung einer Kassette (9) mit einem Deckplatte-Feder-Ventilmechanismus für den notwendigen manuellen Tracheostomaverschluss zum Sprechen angebracht ist.

2. Lätzchen (1) gemäss Anspruch 1, **dadurch gekennzeichnet, dass** sämtliche Partien, die direkt mit der Haut in Berührung kommen und zum Schutze der darüber- und darunterliegenden Kleider und Wächestücke aus einem hautfreundlichen, feinen und weichen Textilgewebe (10) bestehen.

3. Lätzchen (1) gemäss Anspruch 1, **dadurch gekennzeichnet, dass** das hautfreundliche, steife und luftdurchlässigen Textilgewebe (4) an den Rändern zur Schonung der Haut, der Kleider und Wäschestücke mit einem hautfreundlichen, feinen und weichen Textilgewebe (11) eingesäumt ist.

4. Lätzchen (1) gemäss Anspruch 1, **dadurch gekennzeichnet, dass** die beiden Seiten (12 und 13) der auswechselbaren Tasche (7) je aus mindestens einer Lage aus einem hautfreundlichen, feinen und weichen Textilgewebe (14) bestehen.

5. Lätzchen (1) gemäss Anspruch 4, **dadurch gekennzeichnet, dass** die beiden Seiten (12 und 13) der auswechselbaren Tasche (7) zusätzlich je aus einer weiteren Lage Textilgewebe (15) bestehen und deren Zwischenräume mit Füllstoff (16) als Polster ausgefüllt werden.

6. Lätzchen (1) gemäss Anspruch 5, **dadurch gekennzeichnet, dass** die beiden Seiten (12 und 13) der auswechselbaren Tasche (7) je mit einem der Form der Kassette (9) entsprechenden Durchbruch (17) versehen sind.

7. Lätzchen (1) gemäss Anspruch 6, **dadurch gekennzeichnet, dass** der Rand des Durchbruches (17) der auswechselbaren Tasche (7) auf der dem Körper zugewandten Seite (13) mit einem Wulst (18) zur besseren manuell vorzunehmenden Abdichtung des Tracheostomas beim Sprechen versehen ist, welcher aus einem weichen und elastischen Material (20) besteht und mit einem hautfreundlichen, feinen und weichem Textilgewebe (19) eingesäumt ist.
